# EUROPÄISCHE PATENTANMELDUNG

(11) **EP 4 555 923 A1**
(43) Veröffentlichungstag der Anmeldung: **21.05.2025**
(21) Anmeldenummer: 24210225.9
(22) Anmeldetag: 31.10.2024
(51) Int. Cl.: A61B 5/00

(54) **UNTERSUCHUNGSGERÄT FÜR OPTISCHE MEDIZINISCHE UNTERSUCHUNGEN**

(30) Priorität: 10.11.2023 DE 102023131275
(71) Anmelder: HEINE Optotechnik GmbH & Co. KG, 82205 Gilching (DE)
(72) Erfinder: MICHEL, Felix, 82205 Gilching (DE); HEIN, Holger, 82205 Gilching (DE)
(74) Vertreter: Flach Bauer & Partner Patentanwälte mbB

(57) **Zusammenfassung**

Ein Untersuchungsgerät (10) für optische medizinische Untersuchungen, insbesondere ein Dermatoskop, hat ein Gehäuse (12), einen Prozessor (42) und einen Wärmeableiter (44), wobei das Gehäuse (12) einen Innenraum umschließt, wobei der Prozessor (42) im Innenraum angeordnet ist. Der Wärmeableiter (44) weist einen Wärmequellenabschnitt (56) und einen Wärmesenkenabschnitt (58) auf, wobei der Wärmequellenabschnitt (56) im Innenraum angeordnet ist und mit dem Prozessor (42) in thermischem Kontakt steht, und wobei zumindest eine Seite des Wärmesenkenabschnitts (58) außerhalb des Innenraums angeordnet ist.

## Beschreibung

Die Erfindung betrifft ein Untersuchungsgerät für optische medizinische Untersuchungen, insbesondere ein Dermatoskop.

Untersuchungsgerät für optische medizinische Untersuchungen, insbesondere Dermatoskope, die eine Kamera zur Untersuchung und einen Bildschirm zur Darstellung der Aufnahmen der Kamera aufweisen, sind bekannt. Solche Untersuchungsgeräte bieten gegenüber rein optischen Untersuchungsgeräten den Vorteil, dass während der Untersuchungen Aufnahmen gemacht und dokumentiert werden können.

Wünschenswert wäre es jedoch, dass das Untersuchungsgerät den Bediener bei der Untersuchung unterstützt, indem weitergehende Informationen auf dem Bildschirm während der Untersuchung angezeigt werden.

Um solche Funktionalität bereitstellen zu können, sind jedoch leistungsfähige Prozessoren mit Neuromorphen Prozessoren, mit Grafikprozessoren und/oder mit Gleitkommaeinheiten im Untersuchungsgerät notwendig. Solche leistungsstarken Prozessoren erzeugen allerdings während des Betriebs eine große Menge an Wärme, die vom Prozessor abgeführt und in die Umgebung abgeleitet werden muss.

Dies ist jedoch bei einem medizinischen Untersuchungsgerät problematisch, da bekannte Lösungen zum Abtransport von Abwärme, wie Lüftungsschlitze, die notwendige gründliche Reinigung oder eine Desinfektion verhindern oder erheblich erschweren.

Die Aufgabe wird gelöst durch ein Untersuchungsgerät für optische medizinische Untersuchungen, insbesondere ein Dermatoskop. Das Untersuchungsgerät weist ein Gehäuse, einen Kopfbereich, einen Griffbereich, einen Prozessor, eine Kamera, einen Bildschirm und einen Wärmeableiter auf, wobei das Gehäuse einen Innenraum umschließt, und der Prozessor im Innenraum angeordnet ist. Die Kamera und der Bildschirm sind im Kopfbereich angeordnet. Der Wärmeableiter weist einen Wärmequellenabschnitt und einen Wärmesenkenabschnitt auf, wobei der Wärmequellenabschnitt im Innenraum angeordnet ist und mit dem Prozessor in thermischem Kontakt steht. Zumindest eine Seite des Wärmesenkenabschnitts ist außerhalb des Innenraums angeordnet.

Die Erfinder haben erkannt, dass die Abwärme des Prozessors dadurch zuverlässig und sicher abgeleitet werden kann, dass ein Wärmesenkenabschnitt außerhalb des Innenraums angeordnet ist. Hierdurch ist es nicht notwendig, dass das Gehäuse Lüftungsschlitze oder dergleichen aufweist, die eine medizinische Reinigung und/oder Desinfektion und somit eine Aufbereitung erschweren oder verhindern würden. Außerdem werden durch den Kopfbereich und den Griffbereich dezidierte Bereiche am Untersuchungsgerät vorgesehen und die Ergonomie vergrößert.

Die zumindest eine Seite des Wärmesenkenabschnitt, die nicht im Innenraum angeordnet ist, steht insbesondere mit der Umgebung des Gehäuses in Kontakt und/oder ist nicht vom Gehäuse umschlossen.

Der Wärmeableiter stellt zum Beispiel eine wärmeableitende Verbindung mittel Wärmebrücken zwischen dem Wärmequellenabschnitt und dem Wärmesenkenabschnitt bereit.

Der Innenraum kann mehrere Unterräume haben.

Das Untersuchungsgerät ist bevorzugt ein dezidiertes Gerät, also ein für diesen Zweck entworfenes Gerät. Insbesondere ist das Untersuchungsgerät kein Telefon, kein Smartphone, kein Tablet oder kein sonstiges Smart Device.

Zum Beispiel ist das Untersuchungsgerät ein Handgerät, d.h. es kann bei der Untersuchung vollständig vom Bediener getragen sein.

In einer Ausgestaltung weist der Prozessor einen Neuromorphen Prozessor (neural processing unit), einen Grafikprozessor (graphics processing unit - GPU) und/oder eine Gleitkommaeinheit (floating-point unit) auf, wodurch eine Bildbearbeitung effizient möglich ist.

Der Prozessor kann als weitere Einheiten einen Hauptprozessor (central processing unit - CPU) und/oder eine Displayschnittstelle aufweisen.

Beispielsweise wird der thermische Kontakt zwischen dem Wärmequellenabschnitt und dem Prozessor durch unmittelbaren physischen Kontakt oder Kontakt mittelbaren physischen Kontakt mittels nur einem Wärmeleitmaterial (im Englischen auch als "thermal interface material" bezeichnet), insbesondere einer Wärmeleitpaste und/oder einer Wärmeleitfolie erzeugt.

In einem Aspekt bildet der Wärmesenkenabschnitt einen Teil der Außenfläche des Untersuchungsgeräts, insbesondere des Gehäuses, wodurch das Untersuchungsgerät noch einfacher zu reinigen ist.

Denkbar ist auch, dass der Wärmesenkenabschnitt außerhalb des Gehäuses angeordnet ist.

In einer Ausführungsform ist der Wärmesenkenabschnitt am Kopfbereich und/oder am Griffbereich angeordnet.

Zum Beispiel ist der Prozessor am Übergang vom Kopfbereich zum Griffbereich angeordnet, um den Prozessor optimal anbinden zu können.

Um den Komfort zu erhöhen und die Sicherheit in Bezug auf vorgeschriebene Berührtemperaturen zu gewährleisten, kann das Untersuchungsgerät eine Patientenseite und eine Bedienerseite aufweisen, wobei der Wärmesenkenabschnitt nicht auf der Patientenseite und/oder nicht auf der Bedienerseite angeordnet ist, und/oder das Untersuchungsgerät kann mehrere laterale Seiten haben, wobei der Wärmesenkenabschnitt auf wenigstens einer der lateralen Seiten angeordnet ist.

Es können mehrere Wärmesenkenabschnitte vorgesehen sein. Insbesondere können die Wärmesenkenabschnitte an verschiedenen lateralen Seiten vorgesehen sein.

Für eine besonders effiziente Wärmeleitung kann der Wärmeableiter einstückig oder als einstückiger Träger mit einem Einleger ausgeführt sein, und/oder der Wärmeableiter ist aus Metall, insbesondere aus Kupfer, Aluminium oder einer Legierung hiervon hergestellt.

In einer Ausgestaltung ist der einstückige Träger aus einem ersten Metall, zum Beispiel einer Aluminiumlegierung, und der Einleger ist aus einem zweiten Metall, zum Beispiel Kupfer. Der Einleger ist im Träger eingelassen, zum Beispiel im Wärmequellenabschnitt. Denkbar ist, dass der Einleger den Wärmequellenabschnitt bildet.

Beispielsweise geht der Wärmequellenabschnitt in die Wärmebrücke über und die Wärmebrücke geht in den Wärmesenkenabschnitt über.

In einer Ausführungsform weist das Untersuchungsgerät einen Rahmen auf, der einen Teil der Außenfläche des Gehäuses bildet, wobei der Wärmesenkenabschnitt ein Teil des Rahmens ist. Auf diese Weise kann der Rahmen, der die Stabilität des Untersuchungsgeräts bereitstellt, gleichzeitig als Wärmesenke verwendet werden.

Zur weiteren Steigerung der Wärmeableitung können der Rahmen und der Wärmeableiter, insbesondere sein einstückiger Träger, zusammen einstückig ausgeführt sein.

Um die Wärme definiert in den Rahmen einzuleiten, kann der Wärmeableiter eine Wärmebrücke aufweisen, die den Wärmequellenabschnitt mit dem Wärmesenkenabschnitt thermisch verbindet, und die Wärmebrücke kann den Rahmen an einem Kontaktpunkt physisch kontaktieren, insbesondere in den Rahmen übergehen.

Der Rahmen kann die Kontur des Untersuchungsgeräts an den lateralen Seiten umlaufen, um besonders stabil zu sein, und/oder wenigstens eine Unterbrechung aufweisen, um den Wärmefluss steuern zu können.

Die Unterbrechung ist beispielsweise kleiner als 1 cm, insbesondere kleiner als 0,5 cm in Längsrichtung, um die Stabilität des Rahmens nicht zu beeinträchtigen. Der Rahmen kann die Kontur bis auf diese eine oder mehrere Unterbrechungen vollständig umlaufen.

In einer Ausgestaltung ist eine Griffzone am Untersuchungsgerät, insbesondere am Griffbereich vorgesehen, insbesondere wobei die Griffzone zwischen einer Unterbrechung des Rahmens und einem Kontaktpunkt des Rahmens angeordnet ist oder zwischen zwei Unterbrechungen angeordnet ist. Auf diese Weise wird sichergestellt, dass sich die Griffzone nur langsam erwärmt und Berührtemperaturen sicher eingehalten werden.

Es ist denkbar, dass die Griffzone gummiert oder anderweitig bedeckt ist, um sie für ein verbessertes haptisches Gefühl thermisch zu entkoppeln.

Beispielsweise kann im Bereich der Griffzone ein Griffstück vorgesehen sein, das beabstandet von der Griffzone ausgeführt ist und die Griffzone abdeckt.

Der Kontaktpunkt ist insbesondere auf der vom Wärmequellenabschnitt abgewandten Seite der Griffzone angeordnet.

Für eine einfache Konstruktion des Gehäuses kann das Gehäuse zwei Gehäuseschalen aufweisen, die insbesondere am Rahmen befestigt sind.

Um den Komfort und die Sicherheit des Patienten zu gewährleisten, kann das Untersuchungsgerät auf der Patientenseite an der Außenfläche eine Auflagezone aufweisen, wobei die Auflagezone thermisch vom Wärmeableiter und/oder vom Rahmen getrennt ist.

In einer Ausgestaltung ist die Kamera auf der Patientenseite angeordnet, und/oder die Kamera weist ein Makroobjektiv auf, und/oder der Bildschirm ist auf der Bedienerseite angeordnet; und/oder das Untersuchungsgerät weist eine Benutzerschnittstelle, insbesondere wenigstens eine Taste, auf, die im Griffbereich und/oder auf der Bedienerseite angeordnet ist.

Die Kamera kann am Rahmen befestigt sein.

Es kann eine zweite Kamera vorgesehen sein, die nach vorne gerichtet ist, insbesondere wobei die Blickfelder der beiden Kameras sich nicht überlappen.

In einer Ausgestaltung ist der Prozessor dazu ausgebildet, die von der erste und/oder zweiten Kamera aufgenommene Aufnahme zu empfangen, zu verarbeiten, insbesondere eine Bildanalyse durchzuführen, und/oder den Bildschirm anzusteuern, um die Aufnahme und insbesondere die Ergebnisse der Bildanalyse in Echtzeit anzuzeigen. Auf diese Weise können weitergehende Funktionalitäten bereitgestellt werden, die den Bediener bei der Untersuchung unterstützen.

Die Aufnahme ist insbesondere eine Videoaufnahme. Die Untersuchung kann somit wie bei einem rein optischen Gerät erfolgen.

Der Prozessor kann wenigstens zwei Einheiten aufweisen, wobei eine Einheit dazu ausgebildet ist, den Bildschirm anzusteuern. Eine andere Einheit kann dazu ausgebildet sein, die Aufnahme zu verarbeiten, insbesondere die Bildanalyse durchzuführen. Diese Einheit zur Verarbeitung der Aufnahme kann ein Neuromorpher Prozessor und/oder eine Gleitkommaeinheit sein.

Die Einheiten können räumlich getrennt voneinander angeordnet sein, wobei wenigstens eine Einheit, insbesondere die Einheit zur Bildverarbeitung, mit dem Wärmequellenabschnitt in thermischem Kontakt steht.

Das Untersuchungsgerät kann einen integrierten Energiespeicher aufweisen, insbesondere der im Griffbereich angeordnet ist.

Weitere Merkmale und Vorteile der Erfindung ergeben sich aus der nachfolgenden Beschreibung sowie aus den beigefügten Zeichnungen, auf die Bezug genommen wird. In den Zeichnungen zeigen:
- Fig. 1: ein Untersuchungsgerät gemäß einer Ausführungsform der Erfindung in einer perspektivischen Ansicht von vorne,
- Fig. 2: eine Seitenansicht auf eine laterale Seite des Untersuchungsgeräts gemäß Figur 1,
- Fig. 3: eine perspektivische Ansicht des Untersuchungsgeräts gemäß Figur 1 von unten, wobei das Untersuchungsgerät geöffnet ist, und
- Fig. 4: einen einstückigen Rahmen und Wärmeableiter des Untersuchungsgeräts gemäß Figur 3.

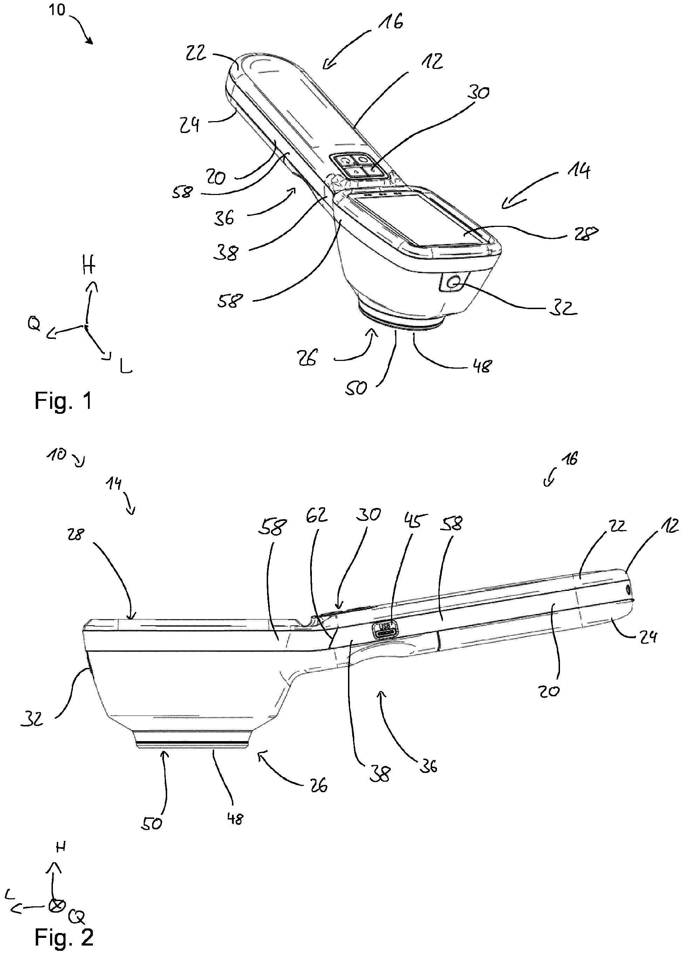

In Figur 1 ist ein Untersuchungsgerät 10 für optische medizinische Untersuchungen perspektivisch in einer Ansicht von vorne oben dargestellt. Zu sehen ist daher überwiegend die Oberseite des Untersuchungsgeräts 10.

Figur 2 zeit das Untersuchungsgerät 10 in einer orthogonalen Seitenansicht auf eine der lateralen Seiten in Querrichtung Q.

Das Untersuchungsgerät 10 ist beispielsweise ein Dermatoskop und ist in der gezeigten Ausführungsform als Handgerät ausgeführt.

Insbesondere ist das Untersuchungsgerät 10 ein dezidiertes medizinisches Untersuchungsgerät bzw. Dermatoskop und beispielsweise für den Dauereinsatz ausgelegt. Zum Beispiel ist das Untersuchungsgerät 10 kein Telefon, Smartphone, Tablet oder sonstiges Smart Device.

Das Untersuchungsgerät 10 weist ein Gehäuse 12 auf und verfügt über einen Kopfbereich 14 und einen Griffbereich 16, der an den Kopfbereich 14 angrenzt.

Das Untersuchungsgerät 10 hat eine Längsrichtung L, eine Querrichtung Q sowie eine Hochrichtung H.

In Hochrichtung H hat das Untersuchungsgerät 10 eine Patientenseite (in Figur 1 die untere Seite) und eine Bedienerseite (in Figur 1 die Oberseite), die zueinander entgegengesetzt sind. Die übrigen Seiten des Untersuchungsgeräts 10 werden als laterale Seiten bezeichnet.

Der Griffbereich 16 erstreckt sich in Längsrichtung L und der Kopfbereich 14 schließt auf der vorderen Seite des Griffbereichs 16 an diesen an.

Der Kopfbereich ist in Längsrichtung L beispielsweise kürzer als der Griffbereich 16.

Der Kopfbereich 14 ins in Hochrichtung H dicker, insbesondere mehr als doppelt so dick wie der Griffbereich 16 ausgebildet. Beispielsweise geht die Oberseite des Griffbereichs 16 in die Oberseite des Kopfbereichs 14 über, wobei die Unterseite des Kopfbereichs 14 versetzt zur Unterseite des Griffbereichs 16 liegt.

In der Querrichtung Q kann der Kopfbereich 14 breiter ausgeführt sein als der Griffbereich 16.

Das Untersuchungsgerät 10 hat einen Rahmen 20 und zwei Gehäuseschalen, nämlich eine obere Gehäuseschale 22 und eine untere Gehäuseschale 24.

Der Rahmen 20, die obere Gehäuseschale 22 und/oder die unter Gehäuseschale 24 erstrecken sich sowohl im Kopfbereich 14 als auch im Griffbereich 16.

Die Gehäuseschalen 22, 24 und Teile des Rahmens 20 sind Teil des Gehäuses 12, insbesondere bilden sie das Gehäuse 12.

Die Außenfläche des Gehäuses 12, d.h. die von der Umgebung des Untersuchungsgeräts 10 zugänglichen Oberflächen des Gehäuses 12, sind die Außenflächen der Gehäuseschalen 22, 24 und die Außenfläche des Rahmens 20.

Beispielsweise ist obere Gehäuseschale 22 an der Oberseite des Rahmen 20 und die untere Gehäuseschale 24 an der Unterseite des Rahmens 20 befestigt. Insbesondere sind die Außenflächen der Gehäuseschalen 22, 24 durch die Außenfläche des Rahmens 20 voneinander beabstandet, wie in Figur 2 gut zu erkennen ist.

Das Gehäuse 12 begrenzt einen Innenraum des Untersuchungsgeräts 10, in dem weitere Komponenten des Untersuchungsgeräts 10 angeordnet sind. Der Innenraum kann in verschiedene Unterräume unterteilt sein, in denen verschiedene der Komponenten untergerbacht sind.

Das Untersuchungsgerät 10 weist wenigstens eine Kamera 26, einen Bildschirm 28 und eine Benutzerschnittstelle 30 auf, die von außerhalb des Gehäuses 12 bzw. der Umgebung des Untersuchungsgeräts 10 zugänglich sind.

Im gezeigten Ausführungsbeispiel weist das Untersuchungsgerät eine erste Kamera 26 und zudem eine zweite Kamera 32 auf, die von außerhalb des Gehäuses bzw. der Umgebung des Untersuchungsgeräts 10 zugänglich sind.

Im Kopfbereich 14 sind die erste Kamera 26, die zweite Kamera 32 und der Bildschirm 28 angeordnet.

Die erste Kamera 26 ist an der Patientenseite des Kopfbereichs 14 angeordnet und hat ein Blickfeld durch die Außenfläche an der Unterseite nach unten.

Die zweite Kamera 32 ist vorne im Kopfbereich 14 angeordnet und hat ein Blickfeld durch die vordere laterale Seite des Untersuchungsgerätes 10.

Die Blickfelder der ersten Kamera 26 und der zweiten Kamera 32 sind somit frei von Überlapp.

Der Bildschirm 28 ist auf der Bedienerseite angeordnet und bedeckt den Großteil, insbesondere 80% der Bedienerseite des Kopfbereichs 14.

Der Griffbereich 16 weist die Benutzerschnittstelle 30 auf, die in Form von Tasten an der Bedienerseite des Griffbereichs 16 angeordnet sind. Es ist denkbar, dass die Benutzerschnittstelle 30 auch anders ausgestaltet ist, beispielsweise als berührungsempfindliches Display und/oder als Teil des Bildschirms 28.

Entlang des Griffbereichs 16 ist die Benutzerschnittstelle 30 an dem dem Kopfbereich 14 zugewandten Ende des Griffbereichs 16 angeordnet, sodass diese von einer Bedienperson des Untersuchungsgeräts 10 beispielsweise mit dem Daumen bedient werden können, wenn diese den Griffbereich 16 mit der Hand ergreift.

An einer Patientenseite des Griffbereichs 16 ist zudem ein Verjüngungsabschnitt 36 vorhanden, der derart ausgeformt ist, dass die Bedienperson beispielsweise den Zeigefinger in den Verjüngungsabschnitt 36 einlegen kann, wenn der Griffbereich 16 mit der Hand ergriffen wird. Auf diese Weise ergibt sich eine besonders ergonomische Handhaltung bei der Benutzung des Untersuchungsgeräts 10.

An den lateralen Seiten ist im Griffbereich 16 wenigstens eine Griffzone 38 am Rahmen 20 vorgesehen, an der ein Teil der Hand der Bedienperson anliegt, wenn der Griffbereich 16 mit der Hand ergriffen wird.

Die Griffzone 38 liegt beispielsweise in Längsrichtung zumindest im Bereich des Verjüngungsabschnitt 36, im Bereich der Benutzerschnittstelle 30 und/oder an dem dem Kopfbereich 14 zugewandten Ende des Griffbereichs 16.

Beispielsweise sind zwei Griffzonen 38 an einander gegenüberliegenden lateralen Seiten vorhanden.

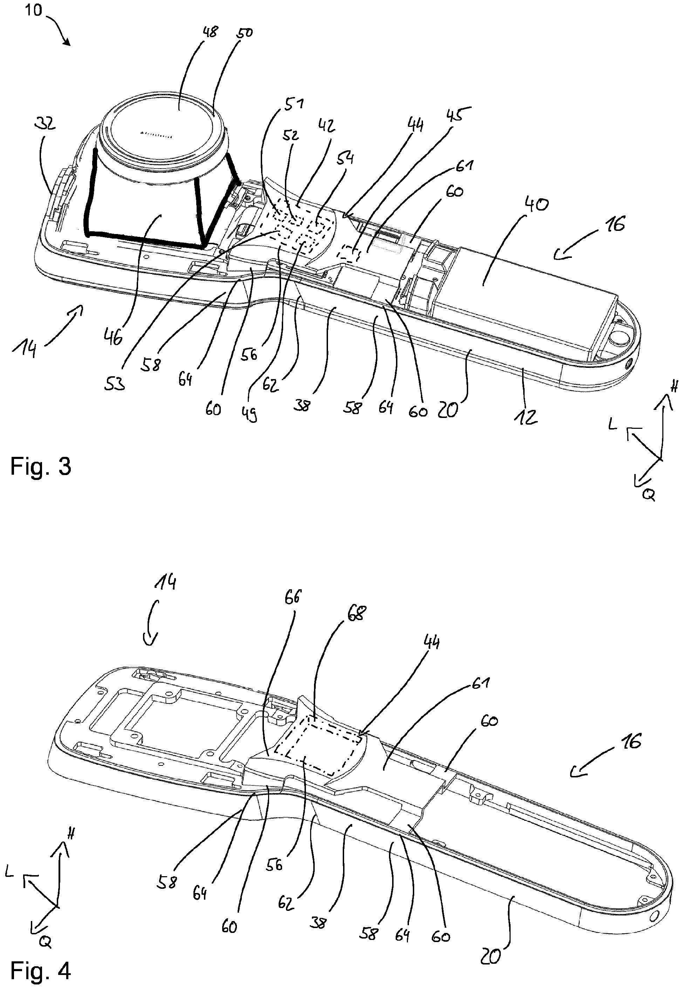

Figur 3 zeigte das Untersuchungsgerät 10 in einer perspektivischen Ansicht von unten und leicht von hinten, wobei die untere Gehäuseschale 24 entfernt ist.

Neben der ersten Kamera 26 und der zweite Kamera 32 sind weitere Komponenten des Untersuchungsgeräts 10 zu erkennen, nämlich ein Energiespeicher 40, ein Prozessor 42, ein Wärmeableiter 44 sowie eine Kommunikationsschnittstelle 45.

In Figur 3 ist zu erkennen, dass die erste Kamera 26 ein Makroobjektiv 46 aufweist und daher als Makrokamera ausgebildet ist.

Zudem hat die erste Kamera 26 ein Deckglas 48, das während der Untersuchung auf dem Patienten aufgelegt werden kann und somit eine Auflagezone 50 bildet.

Die erste Kamera 26 kann am Rahmen 20 befestigt sein.

Die integrierte Kommunikationsschnittstelle 45 ist beispielsweise drahtlos ausgeführt, wie ein Bluetooth-Modul oder ein WLAN-Modul. Aber auch eine kabelgebundene Kommunikationsschnittstelle 45, beispielsweise in Form eines USB-Anschlusses (Fig. 2), sind denkbar.

Der Energiespeicher 40 ist im Griffbereich 16 angeordnet, insbesondere am hinteren Ende in Längsrichtung L, d.h. an dem vom Kopfbereich 14 abgewandten Ende des Griffbereichs 16.

Auf diese Weise kann der Energiespeicher 40 zugleich als Ausgleichsmasse innerhalb des Untersuchungsgeräts 10 dienen, um das Gewicht des Kopfbereichs 14 wenigstens teilweise auszutarieren, wodurch das Untersuchungsgerät 10 besonders präzise und mit wenig Anstrengung von der Bedienperson geführt werden kann.

Der Energiespeicher 40 ist ein wiederaufladbarer Akkumulator.

Beispielsweise ist das Untersuchungsgerät 10 Teil einer Untersuchungsgerätebaugruppe, die das Untersuchungsgerät 10 und eine Ladestation für das Untersuchungsgerät 10 umfasst. Die Ladestation kann in diesem Fall eine Aufnahme aufweisen, in die das Untersuchungsgerät 10 mit dem eine Ladeschnittstelle aufweisenden Ende des Griffbereichs 16 (hier das hintere Ende) zerstörungsfrei lösbar eingesteckt wird, um einen Ladevorgang durchzuführen.

Grundlegend ist es auch möglich, dass anstelle eines wiederaufladbaren Energiespeichers 40 eine einmalig nutzbare Batterie als austauschbarer Energiespeicher 40 vorhanden ist.

Der Prozessor 42 ist zwischen der ersten Kamera 26 und dem Energiespeicher 40 in Längsrichtung L angeordnet.

Beispielsweise ist der Prozessor 42 am Übergang vom Kopfbereich 14 zum Griffbereich 16 angeordnet. Zum Beispiel ist der Prozessor in Längsrichtung im Bereich der Griffzone 38 platziert oder nach vorne versetzt zur Griffzone 38.

Der Prozessor 42 und der Energiespeicher 40 sind vollständig innerhalb des Gehäuses 12, d. h. vollständig im Innenraum angeordnet.

Der Prozessor 42 ist dazu ausgebildet, die von der ersten Kamera 26 und/oder der zweiten Kamera 32 aufgenommene Aufnahme zu empfangen und den Bildschirm 28 derart anzusteuern, dass diese Aufnahme auf dem Bildschirm 28 dargestellt wird.

Die Aufnahme ist insbesondere eine Videoaufnahme, die in Echtzeit auf dem Bildschirm 28 wiedergegeben wird.

Der Prozessor 42 ist weiterhin dazu ausgebildet, die Aufnahmen nicht nur zu empfangen und weiterzuleiten, sondern auch zu verarbeiten, beispielsweise eine Bildanalyse in Echtzeit durchzuführen.

Während der Bildanalyse führt der Prozessor 42 zum Beispiel eine Objekt- bzw. Kantenerkennung in der aufgenommenen Aufnahme durch. Im Falle eines Dermatoskops kann der Prozessor 42 mittels der Bildanalyse Melanome erkennen, verschiedene Bereiche der Melanome vermessen und/oder die Melanome kategorisieren.

Als Ergebnis der Bildanalyse kann der Prozessor 42 beispielsweise Umrisse von Strukturen, wie Melanome, in der Aufnahme markieren und/oder eine Risikobewertung ausgeben.

Der Bildschirm 28 wird derart vom Prozessor 42 gesteuert, dass die Ergebnisse der Bildanalyse in Echtzeit gleichzeitig mit der aufgenommenen Aufnahme, insbesondere mit der Aufnahme überlagert, auf dem Bildschirm 28 dargestellt werden.

Der Prozessor 42 kann aus wenigstens zwei Einheiten bestehen, wobei eine Einheit dazu ausgebildet ist, die Aufnahme zu verarbeiten, insbesondere die Bildanalyse durchzuführen, und/oder eine Einheit dazu ausgebildet ist, den Bildschirm anzusteuern.

Die Einheiten können auf einem einzigen Chip oder räumlich getrennt voneinander angeordnet sein.

Um die Bildanalyse, insbesondere in Echtzeit durchführen zu können, weist der Prozessor 42 beispielsweise als Einheit einen Neuromorphen Prozessor 52 (neural processing unit), einen Grafikprozessor 51 (graphics processing unit - GPU) und/oder eine Gleitkommaeinheit 54 (floating point unit) auf.

Der Prozessor 42 kann als weitere Einheit einen Hauptprozessor 53 (central processing unit - CPU) und/oder eine Displayschnittstelle aufweisen.

Um die beim Betrieb des Prozessors 42 entstehende Wärme abzuleiten, ist der Wärmeableiter 44 vorgesehen.

Der Wärmeableiter 44 weist einen Wärmequellenabschnitt 56, einen Wärmesenkenabschnitt 58 sowie eine Wärmebrücke 60 auf.

Im gezeigten Ausführungsbeispiel hat der Wärmeableiter 44 einen Wärmequellenabschnitt 56, vier Wärmesenkenabschnitte 58 und vier Wärmebrücken 60.

Die Wärmebrücken 60 verbindenden den Wärmequellenabschnitt 56 mit den Wärmesenkenabschnitten 58 thermisch und stellen so eine wärmeableitende Verbindung bereit.

Die Auflagezone 50 ist insbesondere thermisch vom Wärmeableiter 44 und dem Rahmen 20 getrennt.

Der Wärmeableiter 44 ist einstückig ausgeführt, d.h. dass der Wärmequellenabschnitt 56, der Wärmesenkenabschnitt 58 und die Wärmebrücken 60 Teil des gleichen Werkstücks sind.

Der Wärmequellenabschnitt 56 geht somit in die Wärmebrücken 60 über, die wiederum in die jeweiligen Wärmesenkenabschnitte 58 übergehen.

Zum Beispiel besteht der Wärmeableiter 44 aus einem einzigen Werkstück aus Metall, insbesondere aus Kupfer, Aluminium, einer Kupferlegierung oder einer Aluminiumlegierung.

Im gezeigten Ausführungsbeispiel sind der Wärmeableiter 44 und der Rahmen 20 zusammen einstückig ausgeführt, d.h. als ein einziges Werkstück, beispielsweise als Werkstück aus Metall. Das Werkstück aus Rahmen 20 und Wärmeableiter 44 ist isoliert in Figur 4 dargestellt.

Zu erkennen ist, dass der Rahmen 20 die Kontur des Untersuchungsgeräts 10 an den lateralen Seiten vollständig umläuft. Als Kontur wird beispielsweise die Außenkontur des Untersuchungsgeräts 10 in einer orthogonalen Ansicht von der Bedienerseite angesehen.

Der Rahmen 20 hat auf jeder der lateralen Seiten in Querrichtung Q im Bereich des Übergangs vom Kopfbereich 14 zum Griffbereich 16 eine Unterbrechung 62. Die Unterbrechungen 62 sind beispielsweise jeweils in Längsrichtung L vor der Griffzone 38 vorgesehen.

Die Unterbrechungen 62 sind in Längsrichtung L jeweils kleiner als 1 cm, insbesondere kleiner als 0,5 cm. Sie können als Schlitze im Rahmen 20 ausgebildet sein.

An den Unterbrechungen 62 stehen die an die Unterbrechung 62 angrenzenden Abschnitte des Rahmen 20 nicht in thermischem Kontakt. Die Unterbrechung 62 verhindert somit die Wärmeleitung durch den Rahmen 20 an dieser Stelle.

Einige Abschnitte des Rahmens 20 bilden die Wärmesenkenabschnitt 58 des Wärmeableiter 44. Die Wärmesenkenabschnitte 58 sind somit Teil des Rahmens 20.

So wie der Rahmen 20 selbst sind die Wärmesenkenabschnitt 58 daher außerhalb des Innenraums angeordnet, zumindest mit ihrer Außenseite. Diese Seite steht daher mit der Umgebung des Gehäuses 12 in Kontakt und ist nicht von Gehäuse 12 umschlossen.

Die Wärmesenkenabschnitte 58 sind somit ein Teil der Außenfläche des Untersuchungsgeräts 10, insbesondere ein Teil der Außenfläche des Gehäuses 12.

Insbesondere fluchtet die zumindest eine Seite des Wärmesenkenabschnitts 58, die außerhalb des Innenraums angeordnet ist, mit dem übrigen Gehäuse 12.

Denkbar ist auch, dass die Wärmesenkenabschnitte 58 vollständig außerhalb des Gehäuses 12 angeordnet sind, beispielsweise vom Gehäuse 12 abstehen.

Im gezeigten Ausführungsbeispiel sind zwei Wärmesenkenabschnitte 58 im Kopfbereich 14 und zwei Wärmesenkenabschnitte 58 im Griffbereich 16 angeordnet. Beispielsweise ist jeweils ein Wärmesenkenabschnitt 58 im Kopf- bzw. Griffbereich 14, 16 auf jeder der lateralen Seiten in Querrichtung Q vorhanden.

Die Wärmesenkenabschnitte 58 sind somit an den lateralen Seiten, hier den lateralen Seiten in Querrichtung Q, angeordnet und insbesondere nicht auf der Bedienerseite oder der Patientenseite.

In Längsrichtung List auf jeder der lateralen Seiten in Querrichtung Q zwischen dem Wärmesenkenabschnitt 58 im Kopfbereich 14 und dem Wärmesenkenabschnitts 58 im Griffbereich 16 eine der Unterbrechungen 62 platziert.

In den Wärmesenkenabschnitten 58 liegen Kontaktpunkte 64 des Rahmens 20, von denen aus sich die Wärmebrücken 60 in den Innenraum erstrecken.

Die Wärmebrücken 60 kontaktieren den Rahmen 20 an den Kontaktpunkten 64 somit physisch.

Die Griffzonen 38 jeder der lateralen Seiten in Querrichtung Q sind zwischen der jeweiligen Unterbrechungen 62 und dem jeweils nächstliegenden Kontaktpunkt 64 des Rahmens 20 angeordnet.

Zwischen der Unterbrechung 62 und der Griffzone 38 ist kein Kontaktpunkt 64 vorhanden.

Die Griffzonen 38 liegen in Längsrichtung L nach hinten von der Unterbrechung 62 und von dem Kontaktpunkt 64 nach vorne versetzt.

Die Wärmebrücken 60, die von den Kontaktpunkten 64 im Kopfbereich 14 ausgehen, erstrecken sich in Querrichtung Q nach innen, bis sie in den Wärmequellenabschnitt 56 übergehen.

Die Wärmebrücken 60, die von den Kontaktpunkten 64 im Griffbereich 16 ausgehen, erstrecken sich zunächst in Querrichtung Q nach innen und anschließend in Längsrichtung L nach vorne, bis sie in den Wärmequellenabschnitt 56 übergehen.

Der in Längsrichtung L verlaufende Abschnitt 61 der Wärmebrücken 60 kann gemeinsam ausgeführt sein.

Der in Längsrichtung L verlaufende Abschnitt 61 der Wärmebrücken 60 führt dazu, dass der Kontaktpunkt 64 im Griffbereich 16 weiter hinten von der Griffzone 38 platziert werden kann, obwohl der Wärmequellenabschnitt 56 und damit der Prozessor 42 in Längsrichtung L vor der Griffzone 38 liegt.

Dies führt zu einem besonders langen Wärmeübertragungsweg, sodass sich die Griffzone 38 nur langsam erwärmt. Der potentiell kürzere Wärmeübertragungsweg über den Kontaktpunkt 64 im Kopfbereich 14 ist aufgrund der Unterbrechung 62 unterbunden.

Denkbar ist auch, dass der Rahmen 20 mehrere Unterbrechungen 62 auf jeder der lateralen Seiten in Querrichtung Q aufweist und die Griffzone 38 zwischen zwei dieser Unterbrechungen 62 angeordnet ist.

Der Wärmequellenabschnitt 56 ist in Querrichtung Q mittig im Rahmen 20 platziert. In Längsrichtung L liegt er vor der Griffzone 38.

Denkbar ist in einer alternativen Ausgestaltung auch, dass der Wärmeableiter 44 zweistückig ausgeführt ist und einen Träger 66 und einen Einleger 68 (in Figur 4 gestrichelt dargestellt) aufweist.

Der Träger 66 ist aus einem ersten Metall, zum Beispiel einer Aluminiumlegierung, einstückig hergestellt, in dem der einstückige Einleger 68 aus einem zweiten Metall, zum Beispiel Kupfer, eingelegt ist.

Der Einleger 68 ist im Wärmequelleabschnitt 56 angeordnet oder bildet diesen. Hierzu kann der der Träger 66 im Wärmequellenabschnitt 56 eine Aussparung aufweisen.

Der Träger 66 entspricht, bis auf die Aussparung für den Einleger 68, dem zuvor beschriebenen Wärmeableiter 44, insbesondere entsprechen der Träger 66 mit dem Einleger 68 zusammen dem zuvor beschriebenen Wärmeableiter 44 vollständig.

Sofern in dieser Offenbarung die Rede davon ist, dass der Wärmeableiter 44 einstückig mit dem Rahmen 20 ausgeführt ist, bedeutet dies für diese Ausgestaltung, dass der Träger 66 einstückig mit dem Rahmen 20 ausgeführt ist.

Wie in Figur 3 dargestellt, ist der Prozessor 42, insbesondere die Einheit zur Bildverarbeitung bzw. der Neuromorphe Prozessor 52 und/oder die Gleitkommaeinheit 54, am Wärmequellenabschnitt 56 vorgesehen und mit diesem in thermischem Kontakt.

Beispielsweise ist der Prozessor 42 (bzw. die entsprechende Einheit, Neuromorpher Prozessor 52 und/oder Gleitkommaeinheit 54) direkt auf dem Wärmequellenabschnitt 56 befestigt, d.h. sie haben unmittelbaren physischen Kontakt.

Denkbar ist auch, dass nur mittelbarer physischer Kontakt mittels ausschließlich einem Wärmeleitmaterial zwischen dem Prozessor 42 (bzw. der entsprechenden Einheit, Neuromorphen Prozessor 52, Grafikprozessor 51 und/oder Gleitkommaeinheit 54) und dem Wärmequellenabschnitt 56 besteht. Das Wärmeleimaterial kann eine Wärmeleitfolie und/oder eine Wärmeleitpaste, wie ein Wärmeleitkleber, sein.

Wärmeleitmaterial wird im Englischen auch als "thermal interface material" bezeichnet.

Während des Betriebs des Prozessors 42 erwärmt sich dieser und die erzeugte Wärme wird vom Wärmequellenabschnitt 56 aufgenommen.

Die Wärme fließt dann vom Wärmequellenabschnitt 56 über die Wärmebrücken 60 zu den Wärmesenkenabschnitten 58.

Dadurch, dass die Wärmesenkenabschnitte 58 außerhalb des Innenraums angeordnet sind und mit der Umgebung des Gehäuses 12 in Kontakt stehen, können diese die Wärme in die Umgebung abstrahlen bzw. werden durch Luftbewegung gekühlt. Auf diese Weise wird der Prozessor 42 zuverlässige gekühlt.

Gleichzeitig kann die Griffzone 38 durch die Wahl der Platzierung der Kontaktpunkte 64 und/oder der Unterbrechungen 62 vor übermäßiger Wärme geschützt werden, obwohl die Griffzone 38 sehr nahe am Prozessor 42 angeordnet ist.

Es ist denkbar, dass die Griffzone 38 gummiert oder anderweitig bedeckt ist, um sie für ein verbessertes haptisches Gefühl thermisch zu entkoppeln.

Beispielsweise kann im Bereich der Griffzone 38 ein Griffstück vorgesehen sein, an dem der Benutzer das Untersuchungsgerät 10 halten kann. Das Griffstück ist beabstandet von der Griffzone ausgeführt und deckt die Griffzone ab, insbesondere vollständig. Der Abstand zwischen der Griffzone 38 und dem Griffstück ist beispielsweise ein Luftspalt und dient zur thermischen Isolierung des Griffstücks. Der Abstand bzw. Luftspalt ist nach Außen vollständig geschlossen.

Das Griffstück kann an dem Gehäuse 12 befestigt werden, beispielsweise durch kleben, oder als Teil einer oder beider Gehäuseschalen 22, 24 ausgeführt sein.

Gleichzeitig kann der Rahmen eine tragende und stabilisierende Funktion des gesamten Untersuchungsgerät 10 übernehmen, sodass dieser eine Doppelfunktion innehaben kann.

Auf diese Weise ist es daher möglich, einen Prozessor 42, der aufgrund seiner Funktion - beispielsweise durch den Neuromorphen Prozessor 52 und/oder die Gleitkommaeinheit 54 - viel Abwärme erzeugt, in einem tragbaren medizinischen Untersuchungsgerät vorzusehen, ohne dass Öffnungen im Gehäuse 12, wie Lüftungsöffnungen, nötig sind. Eine notwendige hygienische Aufbereitung (Reinigung, Desinfektion und/oder Sterilisation), des Untersuchungsgeräts 10 ist somit problemlos möglich.

Außerdem wird durch die Anordnung der Griffzone 38 und der Auflagezone 50 ein hoher Komfort und Sicherheit des Bedieners oder des Patienten erreicht, da maximale Berührtemperaturen zuverlässig eingehalten werden.

## Patentansprüche

1. Untersuchungsgerät (10) für optische medizinische Untersuchungen, insbesondere Dermatoskop, mit einem Gehäuse (12), einem Kopfbereich (14), einem Griffbereich (16), einem Prozessor (42), einer Kamera (26, 32), einem Bildschirm (28) und einem Wärmeableiter (44), wobei das Gehäuse (12) einen Innenraum umschließt, wobei der Prozessor (42) im Innenraum angeordnet ist,
wobei die Kamera (26, 32) und der Bildschirm (28) im Kopfbereich (14) angeordnet sind,
wobei der Wärmeableiter (44) einen Wärmequellenabschnitt (56) und einen Wärmesenkenabschnitt (58) aufweist, wobei der Wärmequellenabschnitt (56) im Innenraum angeordnet ist und mit dem Prozessor (42) in thermischem Kontakt steht, und wobei zumindest eine Seite des Wärmesenkenabschnitts (58) außerhalb des Innenraums angeordnet ist.

2. Untersuchungsgerät (10) nach Anspruch 1, **dadurch gekennzeichnet, dass** der Prozessor (42) einen Neuromorphen Prozessor (52), einen Grafikprozessor (51) und/oder eine Gleitkommaeinheit (54) aufweist und/oder dass der thermische Kontakt zwischen dem Wärmequellenabschnitt (56) und dem Prozessor (42) durch unmittelbaren physischen Kontakt oder Kontakt mittelbaren physischen Kontakt mittels nur einem Wärmeleitmaterial, insbesondere einer Wärmeleitpaste und/oder einer Wärmeleitfolie erzeugt wird.

3. Untersuchungsgerät (10) nach Anspruch 1 oder 2, **dadurch gekennzeichnet, dass** der Wärmesenkenabschnitt (58) einen Teil der Außenfläche des Untersuchungsgeräts (10), insbesondere des Gehäuses (12) bildet oder außerhalb des Gehäuses (12) angeordnet ist.

4. Untersuchungsgerät (10) nach einem der vorhergehenden Ansprüche, **dadurch gekennzeichnet, dass** der Wärmesenkenabschnitt (58) am Kopfbereich (14) und/oder am Griffbereich (16) angeordnet ist.

5. Untersuchungsgerät (10) nach einem der vorhergehenden Ansprüche, **dadurch gekennzeichnet, dass** der Prozessor (42) am Übergang vom Kopfbereich (14) zum Griffbereich (16) angeordnet ist.

6. Untersuchungsgerät (10) nach einem der vorhergehenden Ansprüche, **dadurch gekennzeichnet, dass** das Untersuchungsgerät (10) eine Patientenseite und eine Bedienerseite aufweist, wobei der Wärmesenkenabschnitt (58) nicht auf der Patientenseite und/oder nicht auf der Bedienerseite angeordnet ist, und/oder dass das Untersuchungsgerät (10) mehrere laterale Seiten hat, wobei der Wärmesenkenabschnitt (58) auf wenigstens einer der lateralen Seiten angeordnet, insbesondere einer der lateralen Seiten in Querrichtung (Q) angeordnet ist.

7. Untersuchungsgerät (10) nach einem der vorhergehenden Ansprüche, **dadurch gekennzeichnet, dass** der Wärmeableiter (44) einstückig oder als einstückiger Träger (66) mit einem Einleger (68) ausgeführt ist, und/oder dass der Wärmeableiter (44) aus Metall, insbesondere aus Kupfer, Aluminium oder einer Legierung hiervon hergestellt ist.

8. Untersuchungsgerät (10) nach einem der vorhergehenden Ansprüche, **dadurch gekennzeichnet, dass** das Untersuchungsgerät (10) einen Rahmen (20) aufweist, der einen Teil der Außenfläche des Gehäuses (12) bildet, wobei der Wärmesenkenabschnitt (58) ein Teil des Rahmens (20) ist.

9. Untersuchungsgerät (10) nach Anspruch 8, **dadurch gekennzeichnet, dass** der Rahmen (20) und der Wärmeableiter (44), insbesondere der einstückige Träger (66), zusammen einstückig ausgeführt sind, und/oder
dass der Wärmeableiter (44) eine Wärmebrücke (60) aufweist, die den Wärmequellenabschnitt (56) mit dem Wärmesenkenabschnitt (58) thermisch verbindet, und wobei die Wärmebrücke (60) den Rahmen (20) an einem Kontaktpunkt (64) physisch kontaktiert, insbesondere in den Rahmen (20) übergeht.

10. Untersuchungsgerät (10) nach Anspruch 8 oder 9, **dadurch gekennzeichnet, dass** der Rahmen (20) die Kontur des Untersuchungsgeräts (10) an den lateralen Seiten umläuft und/oder wenigstens eine Unterbrechung (62) aufweist.

11. Untersuchungsgerät (10) nach Anspruch 10, **dadurch gekennzeichnet, dass** eine Griffzone (38) am Untersuchungsgerät (10), insbesondere am Griffbereich (16) vorgesehen ist, insbesondere wobei die Griffzone (38) zwischen einer Unterbrechung (62) des Rahmens (20) und einem Kontaktpunkt (64) des Rahmens (20) angeordnet ist oder zwischen zwei Unterbrechungen (62) angeordnet ist.

12. Untersuchungsgerät (10) nach einem der Ansprüche 8 bis 11, **dadurch gekennzeichnet, dass** das Gehäuse (12) zwei Gehäuseschalen (22, 24) aufweist, die insbesondere am Rahmen (20) befestigt sind.

13. Untersuchungsgerät (10) nach einem der vorhergehenden Ansprüche, **dadurch gekennzeichnet, dass** das Untersuchungsgerät (10) auf der Patientenseite an der Außenfläche eine Auflagezone (50) aufweist, wobei die Auflagezone thermisch vom Wärmeableiter (44) und/oder vom Rahmen (20) getrennt ist.

14. Untersuchungsgerät (10) nach einem der vorhergehenden Ansprüche, **dadurch gekennzeichnet, dass** die Kamera (26, 32) auf der Patientenseite angeordnet ist, und/oder dass die Kamera (26) ein Makroobjektiv (46) aufweist, und/oder dass der Bildschirm (28) auf der Bedienerseite angeordnet ist; und/oder dass das Untersuchungsgerät (10) eine Benutzerschnittstelle (30), insbesondere wenigstens eine Taste, aufweist, die im Griffbereich (16) und/oder auf der Bedienerseite angeordnet ist.

15. Untersuchungsgerät (10) nach Anspruch 14, **dadurch gekennzeichnet, dass** der Prozessor (42) dazu ausgebildet ist, die von der Kamera (26, 32) aufgenommene Aufnahme zu empfangen, zu verarbeiten, insbesondere eine Bildanalyse durchzuführen, und/oder den Bildschirm (28) anzusteuern, um die Aufnahme und insbesondere die Ergebnisse der Bildanalyse in Echtzeit anzuzeigen.
